Europäisches Patentamt

European Patent Office

Office européen des brevets

(19)

(11) Veröffentlichungsnummer: **0 371 369**
**A1**

# (12) EUROPÄISCHE PATENTANMELDUNG

(21) Anmeldenummer: **89121474.4**

(22) Anmeldetag: **20.11.89**

(51) Int. Cl.⁵: **C07C 303/06**

(30) Priorität: **28.11.88 DE 3840092**

(43) Veröffentlichungstag der Anmeldung:
**06.06.90 Patentblatt 90/23**

(84) Benannte Vertragsstaaten:
**ES GR**

(71) Anmelder: Henkel Kommanditgesellschaft auf Aktien
Postfach 1100 Henkelstrasse 67
D-4000 Düsseldorf 1(DE)

(72) Erfinder: **Behler, Ansgar, Dr.**
**Siegfriedstrasse 80**
**D-4250 Bottrop(DE)**
Erfinder: **Wegener, Ingo**
**Am Falder 20**
**D-4000 Düsseldorf 13(DE)**
Erfinder: **Ritterbex, Horst**
**Beedstrasse 44**
**D-4000 Düsseldorf 30(DE)**
Erfinder: **Selen, Faize**
**Braunsberger Strasse, 8**
**D-4000 Düsseldorf 13(DE)**

(54) Konzentrierte, fliessfähige Aniontenside und Verfahren zu ihrer Herstellung.

(57) Konzentrierte, fließfähige Aniontenside mit einem Wassergehalt von < 1, insbesondere von < 0,5 Gew.-%, lassen sich durch Sulfonierung von ungesättigten Fettsäuren mit 16 bis 22 Kohlenstoffatomen mit Schwefeltrioxid bei molaren Verhältnissen von Schwefeltrioxid zu in den Fettsäuren enthaltenen olefinischen Doppelbindungen von 0,8 bis 1,2 : 1, insbesondere von 0,9 bis 1,0 : 1, und Neutralisation der Sulfonierungsprodukte mit Triethanolamin erhalten.

EP 0 371 369 A1

## Konzentrierte, fließfähige Aniontenside und Verfahren zu ihrer Herstellung.

Die Erfindung betrifft konzentrierte, fließfähige Aniontenside mit einem Wassergehalt von < 1, insbesondere von < 0,5 Gew.-%.

Es ist bekannt, daß durch Abmischen von verschiedenen Anion-und Niotensiden hochkonzentrierte, wasserfreie, bei Umgebungstemperatur fließfähige Pasten erhalten werden können. So beschreibt z.B. die US-C 4 477 372 ein wasserfreies Konzentrat aus einem anionischen und einem nichtionischen Tensid, das ein organisches Sulfat oder Sulfonat, neutralisiert mit verzweigten Hydroxyalkylaminen, enthält. Weiterhin beschreibt die EP-A 243 685 eine wasserverdünnbare, konzentrierte, wässrige Tensidzusammensetzung, die ein nichtionisches Tensid, Lösemittel sowie zwei anionische Tenside aus der von Triethanolaminsalzen von linearen Alkylbenzolsulfonaten, Na-Ethoxysulfat und Na-Alkansulfonat gebildeten Gruppe enthalten. Wasserfreie, bei Umgebungstemperatur fließfähige Aniontensidkonzentrate, die nur eine tensidische Komponente enthalten, sind bisher nicht bekannt geworden.

Die GB-C 1 278 421 beschreibt sulfonierte Fettsäuren, die mit wässrigen Basen, z.B. wässrigem Triethanolamin, neutralisiert und hydrolysiert werden können. Entsprechende Wasser enthaltende Verbindungen sind aus der US-C 2 743 288 bekannt.

Die Erfindung beruht auf der Erkenntnis, daß wasserfreie Pasten eines Triethanolammoniumsalzes von sulfonierten, ungesättigten $C_{16}$-$C_{22}$-Fettsäuren ohne weitere Zusätze in Form von Verdünnungsmitteln oder anderen Additiven, z.B. nichtionischen Tensiden, bei Umgebungstemperatur fließfähige Aniontensidkonzentrate darstellen. Dies ist um so mehr überraschend, als Triethanolammoniumsalze anderer Aniontenside, die von der Struktur her mit Ölsäuresulfonaten verwandt sind, keine fließfähigen Konzentrate bilden; so stellen z.B. Triethanolammoniumsalze von alpha-Sulfo-$C_{16/18}$-Fettsäuren und Methylestern derselben in wasserfreiem Zustand Pasten dar, die bei Umgebungstemperatur nicht mehr fließfähig sind.

Demgemäß ist die Erfindung auf konzentrierte, bei Umgebungstemperatur fließfähige Aniontenside mit einem Wassergehalt von < 1, insbesondere von < 0,5 Gew.-%, gerichtet, die durch Sulfonierung von ungesättigten Fettsäuren mit 16 bis 22 Kohlenstoffatomen mit Schwefeltrioxid bei molaren Verhältnissen von Schwefeltrioxid zu in den Fettsäuren enthaltenen olefinischen Doppelbindungen von 0,8 bis 1,2 : 1, insbesondere von 0,9 bis 1,0 : 1, und Neutralisation der Sulfonierungsprodukte mit Triethanolamin erhältlich sind.

Zu den erfindungsgemäß einsetzbaren ungesättigten Fettsäuren mit 16 bis 22 Kohlenstoffatomen zählen synthetische Fettsäuren mit entsprechender Struktur, insbesondere jedoch Fettsäuren natürlichen, z.B. pflanzlichen und/oder tierischen Ursprungs, einschließlich technischer Gemische derselben, wie sie üblicherweise in der Tensidchemie eingesetzt werden. Typische Beispiele für derartige Fettsäuren sind Palmitolein-, Öl-, Elaidin-, Gadolein-, Brassidin- und Erucasäure. Vorteilhafterweise verwendet man technische Gemische von Fettsäuren, die mindestens 50, insbesondere mindestens 60 Gew.-% einfach ungesättigte Fettsäuren mit 18 Kohlenstoffatomen enthalten.

Zweckmäßigerweise erfolgt die Sulfonierung der Fettsäuren mit hierfür üblichen technischen Apparaturen, insbesondere vom Typ der Fallfilmreaktoren. Die Sulfonierung erfolgt im allgemeinen in Abwesenheit von Lösemitteln; falls besondere Umstände, z.B. eine zu hohe Viskosität des Reaktionsgemisches, auftreten, kann jedoch auch in Gegenwart üblicher, unter den Sulfonierungsbedingungen inerter Lösemittel gearbeitet werden. Bevorzugt erfolgt die Sulfonierung bei Temperaturen von 15 bis 90, insbesondere von 40 bis 70 °C, wobei man mit einem mit einem inerten Trägergas verdünnten Schwefeltrioxidstrom mit einem Schwefeltrioxidgehalt von 1 bis 10 Vol.-% arbeitet.

Es ist weiterhin vorteilhaft, die Sulfonierungsprodukte mit Triethanolamin zu neutralisieren, das einen Wassergehalt von weniger als 1, insbesondere von weniger als 0,5 Gew.-% aufweist. Zwar ist der Einsatz von Triethanolamin mit einem etwas höheren Wassergehalt möglich, die erhaltenenen Aniontenside müssen dann jedoch einem zusätzlichen Trocknungsschritt unterworfen werden.

Gemäß einer weiteren vorteilhaften Ausführungsform der Erfindung neutralisiert man die Sulfonierungsprodukte mit einer zu umgesetztem Schwefeltrioxid und den Carboxylgruppen der Fettsäuren äquivalenten Menge an Triethanolamin, wobei es weiterhin vorteilhaft ist, die Sulfonierungsprodukte unmittelbar nach der Sulfonierung in Triethanolamin zu geben. Weiterhin ist es vorteilhaft, das neutralisierte Gemisch aus Sulfonierungsprodukten und Triethanolamin einer z.B. mehrstündigen Wärmebehandlung bei mindestens 60 °C, insbesondere mindestens 80 °C, zu unterziehen, um intermediär bei der Sulfonierung gebildete Reaktionsprodukte, z.B. Sultone, zu zersetzen.

Die Erfindung betrifft weiterhin Verfahren zur Herstellung der oben genannten konzentrierten, fließfähigen Aniontenside mit den oben erläuterten Merkmalen.

Die Erfindung wird im folgenden anhand eines bevorzugten Ausführungsbeispiels näher erläutert.

Beispiel.

Aus Ausgangsmaterial wurde eine technische, handelsübliche Ölsäure mit einer Säurezahl von 198 bis 203 und einer Jodzahl von 80 bis 97 eingesetzt, die die folgende Zusammensetzung (in Gew.-%) aufwies:

| $C_{12}$-$C_{16}$ | 10 % |
|---|---|
| $C_{16}$ ungesättigt | 5 % |
| $C_{17}$ gesättigt | 1 % |
| $C_{18}$ gesättigt | 2 % |
| $C_{16}$ einfach ungesättigt | 5 % |
| $C_{18}$ einfach ungesättigt | 67 % |
| $C_{18}$ zweifach ungesättigt | 12 % |
| $C_{18}$ dreifach ungesättigt | 1 % |
| > $C_{18}$ | 2 %. |

Die Sulfonierung wurde in einem Fallfilmreaktor aus Glas vorgenommen, der im wesentlichen aus einem von einem Heiz- und Kühlmantel umgebenen Rohr von 1100 mm Länge und 6 mm Innendurchmesser bestand. Der Reaktor war am Kopf mit einer Aufgabevorrichtung für das technische Fettsäuregemisch und einem Gaseinleitungsrohr versehen. Gasförmiges Schwefeltrioxid, durch Erhitzen von Oleum erzeugt, wurde in Form einer Mischung mit Stickstoff (5 Vol.-% $SO_3$) eingesetzt.

Das Fettsäuregemisch wurde mit einer konstanten Geschwindigkeit von 550 g/h aufgegeben. Die Zufuhr des Schwefeltrioxid-Stickstoff-Gemisches wurde so eingestellt, daß das Molverhältnis von Schwefeltrioxid zu ungesättigten Doppelbindungen der Fettsäure 0,92 betrug. Mit Hilfe eines Wasserkreislaufs durch den Reaktormantel wurde die Reaktionstemperatur bei 60 °C gehalten.

Beim Verlassen der Reaktionszone wurde das Reaktionsgemisch in einem Becherglas aufgefangen, in dem wasserfreies Triethanolamin in einer Menge, die zur Neutralisation der Sulfon- und Carbonsäuregruppen erforderlich ist, vorgelegt wurde. Anschließend wurde das Produkt zwei Stunden auf 90 °C erwärmt. Das wasserfreie Produkt war bei Raumtemperatur fließfähig und wies die folgenden Kennzahlen auf:

| Sulfonatgehalt: | 90,9 % |
|---|---|
| unsulfierte Bestandteile: | 5,1 % |
| Triethanolammonium-Sulfat: | 4,0 % |
| Wasser: | weniger als 0,1 %. |

## Ansprüche

1. Konzentrierte, fließfähige Aniontenside mit einem Wassergehalt von < 1, insbesondere von < 0,5 Gew.-%, erhältlich durch Sulfonierung von ungesättigten Fettsäuren mit 16 bis 22 Kohlenstoffatomen mit Schwefeltrioxid bei molaren Verhältnissen von Schwefeltrioxid zu in den Fettsäuren enthaltenen olefinischen Doppelbindungen von 0,8 bis 1,2 : 1, insbesondere von 0,9 bis 1,0 : 1, und Neutralisation der Sulfonierungsprodukte mit Triethanolamin.

2. Aniontenside nach Anspruch 1, dadurch gekennzeichnet, daß man die Sulfonierung bei Temperaturen von 15 bis 90, insbesondere von 40 bis 70 °C durchführt.

3. Aniontenside nach Anspruch 1 oder 2, dadurch gekennzeichnet, daß man die Sulfonierung mit mit einem inerten Trägergas verdünnten Schwefeltrioxidstrom mit einem Schwefeltrioxidgehalt von 1 bis 10 Vol.-% durchführt.

4. Aniontenside nach mindestens einem der Ansprüche 1 bis 3, dadurch gekennzeichnet, daß man die Sulfonierungsprodukte mit Wasser in einer Menge von < 1, insbesondere von < 0,5 Gew.-% enthaltendem Triethanolamin neutralisiert.

5. Aniontenside nach mindestens einem der Ansprüche 1 bis 4, dadurch gekennzeichnet, daß man die Sulfonierungsprodukte mit einer zu umgesetztem Schwefeltrioxid und den Carboxylgruppen der Fettsäuren äquivalenten Menge an Triethanolamin neutralisiert.

6. Aniontenside nach mindestens einem der Ansprüche 1 bis 5, dadurch gekennzeichnet, daß man die Sulfonierungsprodukte unmittelbar nach der Sulfonierung in Triethanolamin gibt.

7. Aniontenside nach mindestens einem der Ansprüche 1 bis 6, dadurch gekennzeichnet, daß man das neutralisierte Gemisch aus Sulfonierungsprodukten und Triethanolamin einer Wärmebehandlung bei mindestens 60° C unterzieht.

8. Aniontenside nach mindestens einem der Ansprüche 1 bis 7, dadurch gekennzeichnet, daß man mindestens 50, insbesondere mindestens 60 Gew.-% einfach ungesättigte Fettsäuren mit 18 Kohlenstoffatomen enthaltende technische Gemische von Fettsäuren sulfoniert.

9. Verfahren zur Herstellung von konzentrierten, fließfähigen Aniontensiden mit einem Wassergehalt von < 1, insbesondere von < 0,5 Gew.-%, dadurch gekennzeichnet, daß man ungesättigte Fettsäuren mit 16 bis 22 Kohlenstoffatomen mit Schwefeltrioxid bei molaren Verhältnissen von Schwefeltrioxid zu in den Fettsäuren enthaltenen olefinischen Doppelbindungen von 0,8 bis 1,2 : 1, insbesondere von 0,9 bis 1,0 : 1, sulfoniert und die Sulfonierungsprodukte mit Triethanolamin neutralisiert.

10. Verfahren nach Anspruch 9, dadurch gekennzeichnet, daß man die Sulfonierung bei Temperaturen von 15 bis 90, insbesondere von 40 bis 70° C durchführt.

11. Verfahren nach Anspruch 9 oder 10, dadurch gekennzeichnet, daß man die Sulfonierung mit einem mit einem inerten Trägergas verdünnten Schwefeltrioxidstrom mit einem Schwefeltrioxidgehalt von 1 bis 10 Vol.-% durchführt.

12. Verfahren nach mindestens einem der Ansprüche 9 bis 11, dadurch gekennzeichnet, daß man die Sulfonierungsprodukte mit Wasser in einer Menge von < 1, insbesondere von < 0,5 Gew.-% enthaltendem Triethanolamin neutralisiert.

13. Verfahren nach mindestens einem der Ansprüche 9 bis 12, dadurch gekennzeichnet, daß man die Sulfonierungsprodukte mit einer zu umgesetztem Schwefeltrioxid und den Carboxylgruppen der Fettsäuren äquivalenten Menge an Triethanolamin neutralisiert.

14. Verfahren nach mindestens einem der Ansprüche 9 bis 13, dadurch gekennzeichnet, daß man die Sulfonierungsprodukte unmittelbar nach der Sulfonierung in Triethanolamin gibt.

15. Verfahren nach mindestens einem der Ansprüche 9 bis 14, dadurch gekennzeichnet, daß man das neutralisierte Gemisch aus Sulfonierungsprodukten und Triethanolamin einer Wärmebehandlung bei mindestens 60° C unterzieht.

16. Verfahren nach mindestens einem der Ansprüche 9 bis 15, dadurch gekennzeichnet, daß man mindestens 50, insbesondere mindestens 60 Gew.-% einfach ungesättigte Fettsäuren mit 18 Kohlenstoffatomen enthaltende technische Gemische von Fettsäuren sulfoniert.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | US-A-2 743 288 (W.H.C. RUEGGEBERG et al.)<br>--- | | C 07 C 303/06 |
| A,D | GB-A-1 278 421 (LANKRO CHEMICALS)<br>----- | | |

RECHERCHIERTE SACHGEBIETE (Int. Cl.5)

C 07 C 303/00
C 11 D    1/00

Der vorliegende Recherchenbericht wurde für alle Patentansprüche erstellt

| Recherchenort | Abschlußdatum der Recherche | Prüfer |
|---|---|---|
| DEN HAAG | 19-02-1990 | VAN GEYT J.J.A. |

EPO FORM 1503 03.82 (P0403)